# EUROPÄISCHE PATENTSCHRIFT

(11) **EP 0 438 715 B1**
(45) Veröffentlichungstag und Bekanntmachung des Hinweises auf die Patenterteilung: **01.06.1994**
(21) Anmeldenummer: 90124225.5
(22) Anmeldetag: 14.12.1990
(51) Int. Cl.: C07C 39/42, C07C 37/20, C07C 35/50, C07C 29/38

(54) **1,1-Bicyclo-substituierte fluorhaltige Cyclopentane und Verfahren zu deren Herstellung**
1,1-Bicyclo-substituted fluorinated cyclopentanes and method for their production
Cyclopentanes-1,1-bicyclo-substitués contenant du fluor et procédé pour leur fabrication

(30) Priorität: 24.01.1990 DE 4001932
(43) Veröffentlichungstag der Anmeldung: 31.07.1991
(73) Patentinhaber: BAYER AG, 51368 Leverkusen (DE)
(72) Erfinder: Negele, Michael, Dr., W-5000 Köln 80 (DE); Marhold, Albrecht, Dr., W-5090 Leverkusen 1 (DE)

(56) Entgegenhaltungen:
- EP-A- 438 698
- US-A- 3 148 220
- US-A- 3 185 736
- JOURNAL OF THE AMERICAN CHEMICAL SOCIETY vol. 72, no. 10, 16 October 1950, pages 4879 - 4884; FRED F. HOLUB ET AL.: 'the action of elementary fluorine upon organic compounds.'
- THE JOURNAL OF ORGANIC CHEMISTRY vol. 33, no. 7, July 1968, pages 2692 - 2696; L. G. ANELLO ET AL.: 'perhalo ketones. xv. the preparation and some reactions of perhalocyclopentanones and -hexanones.'

## Beschreibung

Die vorliegende Erfindung betrifft neue 1,1-Diaryl-substituierte fluorhaltige Cyclopentane und Verfahren zu deren Herstellung aus fluorhaltigen Cyclopentanonen und Arylverbindungen.

Auf dem Gebiet fluorhaltiger Cycloalkane die in 1-Position mit zwei cyclischen Substituenten substituiert sind ist bisher lediglich bekannt, daß man im Labormaßstab 1,1-(Di-4-hydroxybenzol)-hexafluorcyclobutan in nur 35,6 %iger Rohausbeute erhalten kann, wenn man Perfluorcyclobutanon-Hydrat mit Phenol in Gegenwart von wenig konzentrierter Essigsäure und viel konzentrierter Schwefelsäure umsetzt (siehe US-PS 3 388 097, Beispiel 1).

Aus der US-PS 3 185 736 ist ein Verfahren zur Umsetzung von Cyclobutanonen mit aromatischen Verbindungen bekannt. Dabei kann gegebenenfalls in Gegenwart von Katalysatoren wie H₂SO₄, BF₃, P₂O₅ etc. gearbeitet werden.

Umsetzungen mit reaktiven 4-Ringsystemen können nicht Umsetzungen mit weniger reaktiven 5-Ringsystemen naheliegen.

Es wurden nun 1,1-Dicyclo-substituierte fluorhaltige Cyclopentane der Formel (I) gefunden 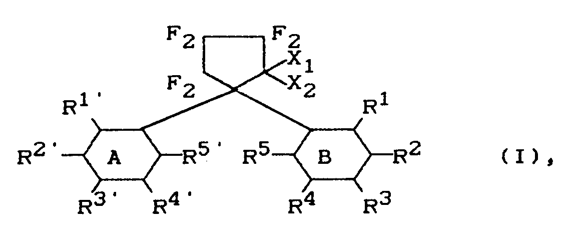
in der
- X₁ und X₂: unabhängig voneinander für Fluor oder Chlor stehen,
- A und B: je für einen Phenylring stehen,
- R¹, R^{1'}, R², R^{2'}, R⁴, R^{4'}, R⁵ und R^{5'}: unabhängig voneinander für Wasserstoff, C₁- bis C₄-Alkyl, Fluor, Chlor oder Brom stehen und
- R³ und R^{3'}: für Hydroxy stehen.

Bevorzugt sind Verbindungen der Formel (I), bei denen X₁ und X₂ gleich sind und die beiden Ringe A und B einschließlich der daran befindlichen Reste ebenfalls übereinstimmen.

Bevorzugt sind weiterhin Verbindungen der Formel (I), bei denen
A und B je einen Phenylrest darstellen und
R¹ und R^{1'} gleich sind und für Wasserstoff oder C₁- bis C₄-Alkyl stehen,
R² und R^{2'} gleich sind und für Wasserstoff, C₁- bis C₄-Alkyl, Fluor, Chlor oder Brom stehen,
R³ und R^{3'} gleich sind und für OH stehen,
R⁴ und R^{4'} gleich sind und für Wasserstoff oder C₁- bis C₄-Alkyl stehen und
R⁵ und R^{5'} für Wasserstoff stehen.

Besonders bevorzugt sind Verbindungen der Formel (I), bei denen X₁ und X₂ für Fluor stehen, A und B je einen Phenylring darstellen, die jeweils nicht besonders angeführten Reste R¹ bis R⁵ und R^{1'} bis R^{5'} für Wasserstoff stehen und
a) R³ = R^{3'} = OH,
b) R¹ = R^{1'} = R⁴ = R^{4'} = Methyl und R³ = R^{3'} = OH,
c) R² = R^{2'} = Methyl und R³ = R^{3'} = OH und
d) R² = R^{2'} = Chlor und R³ = R^{3'} = OH sind.

Bevorzugt ist auch die Verbindung der Formel (I), bei der X₁ und X₂ für Chlor stehen, A und B je einen Phenylring darstellen und R¹, R^{1'}, R², R^{2'}, R⁴, R^{4'}, R⁵ und R⁵' Wasserstoff und R³ und R^{3'} OH sind.

Die vorliegende Erfindung betrifft weiterhin ein Verfahren zur Herstellung von 1,1-Dicyclo-substituierten fluorhaltigen Cyclopentanen der Formel (I), das dadurch gekennzeichnet ist, daß man fluorhaltige Cyclopentanone der Formel (II) 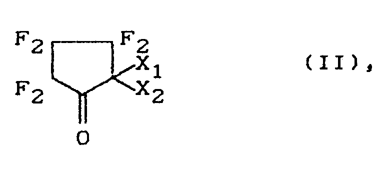
in der
- X₁ und X₂: unabhängig voneinander für Fluor oder Chlor stehen,
mit mindestens der 2-fachen molaren Menge einer oder mehrerer Arylverbindungen der Formel (III) 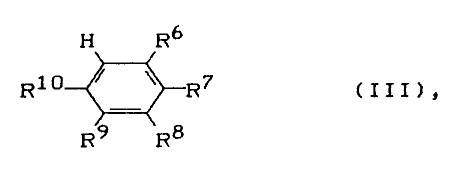
in der
- R⁶ bis R¹⁰: die oben für R¹ bis R⁵ angegebene Bedeutung haben,
in Gegenwart von wasserfreier Flußsäure umsetzt, wobei die Menge wasserfreie Flußsäure so gewählt wird, daß deren Konzentration im Verlauf der Reaktion nicht unter 70 Gew.-% absinkt, und gegebenenfalls anschließend eine Derivatisierung vornimmt.

Als fluorhaltige Cyclopentanone der Formel (II) können Octafluorcyclopentanon, 2-Chlor-heptafluorcyclopentanon und 2,2-Dichlor-hexafluorcyclopentanon eingesetzt werdenk Diese Verbindungen sind z.B. entsprechend J. Org. Chem. 33, 2693 (1968) und der US-PS' en 3 129 248, 3 341 602 und 3 321 515 gut zugänglich.

Bezogen auf 1 Mol eines fluorhaltigen Cyclopentanons der Formel (II) kann man beispielsweise 2 bis 10 Mole einer Arylverbindung der Formel (III) einsetzen. Vorzugsweise beträgt diese Menge 2 bis 2,2 Mole.

Bevorzugt sind Arylverbindungen der Formel (III), bei denen R⁸ für OH steht und R⁶, R⁷, R⁹ und R¹⁰ entweder für Wasserstoff stehen oder einer oder zwei dieser Reste für C₁-bis C₄-Alkyl, Fluor, Chlor oder Brom und die anderen dieser Reste für Wasserstoff stehen.

Besonders bevorzugt als Arylverbindungen der Formel (III) sind Phenol, o-Kresol, 2,6-Dimethylphenol, 2,5-Dimethylphenol und 2-Chlorphenol.

Man kann die wasserfreie Flußsäure auch als Reaktionsmedium verwenden und, bezogen auf das eingesetzte fluorhaltige Cyclopentanon der Formel (II), sie beispielsweise in 50 bis 200-fachem molaren Überschuß einsetzen. Die Menge Flußsäure wird so gewählt, daß ihre Konzentration nicht unter 70 Gew.-% im Verlaufe der Reaktion absinkt, da sonst die korrosive Wirkung auf das Reaktormaterial stark zunimmt. Als wasserfreie Flußsäure ist beispielsweise die im Handel unter dieser Bezeichnung erhältliche Flußsäure geeignet, die z.B. weniger als 1 Gew.-% Wasser enthält.

Als Reaktionstemperaturen kommen beispielsweise solche im Bereich von 20 bis 150°C in Betracht. Bevorzugt sind Temperaturen im Bereich 20 bis 50°C. Die Reaktionszeit kann beispielsweise zwischen 1 und 48 Stunden betragen.

Als Reaktionsgefäße sind beispielsweise Rührkessel aus hochlegierten Stählen geeignet, z.B. solche aus sog. V4A-Stahl. Die Reaktionsgefäße sollten z.B. für Betriebsdrucke bis 150 bar zugelassen sein, damit gegebenenfalls unter Druck gearbeitet werden kann, um die Flußsäure in flüssigem Zustand zu halten. Geeignete Betriebsdrucke liegen, je nach angewendeter Reaktionstemperatur, beispielsweise im Bereich 1 bis 30 bar.

Zur Durchführung des erfindungsgemäßen Verfahrens kann man beispielsweise Flußsäure und das fluorhaltige Cyclopentanon der Formel (II) im Reaktionsgefäß vorlegen und dann die Arylverbindung der Formel (III) zufügen und gegebenenfalls die gewünschte Reaktionstemperatur einstellen. Es ist im allgemeinen vorteilhaft, die Reaktion unter Rühren durchzuführen.

Die 1,1-Dicyclo-substituierten fluorhaltigen Cyclopentane der Formel (I) fallen im allgemeinen in reinerer Form an, wenn nur die stöchiometrisch erforderlichen Mengen oder geringe Überschüsse der Arylverbindung der Formel (III) eingesetzt und relativ niedrige Reaktionstemperaturen, z.B. 20 bis 50°C eingehalten werden.

Das nach der Durchführung des erfindungsgemäßen Verfahrens vorliegende Reaktionsgemisch kann man beispielsweise aufarbeiten, indem man zunächst die Flußsäure, z.B. bei vermindertem Druck, abdestilliert und die erhaltene Verbindung der Formel (I) mit üblichen Methoden isoliert und gegebenenfalls reinigt, z.B. durch Destillieren, Sublimieren, Umfällen und/oder Umkristallisieren.

Auf die bisher beschriebene Weise können beim Einsatz entsprechender Arylverbindungen der Formel (III) Verbindungen der Formel (I) hergestellt werden, bei denen die Ringe A und B identische Phenylringe sind und bei denen die Reste R¹, R^{1'}, R², R^{2'}, R⁴, R^{4'}, R⁵ und R^{5'} unabhängig voneinander Wasserstoff, C₁- bis C₄-Alkyl, Fluor, Chlor oder Brom bedeuten und R³ und R^{3'} für Hydroxy stehen.

Zur Herstellung von Verbindungen der Formel (I), bei denen die Ringe A und B nicht identische Phenylringe sind, sind zusätzliche Maßnahmen erforderlich.

Verbindungen der Formel (I), bei denen A und B zwei verschiedene Phenylringe darstellen, können beispielsweise hergestellt werden, indem man zunächst bezogen auf 1 Mol eines fluorhaltigen Cyclopentanons der Formel (II) nur 0,9 bis 1,1 Mol einer Arylverbindung der Formel (III) einsetzt und so zunächst unter den oben beschriebenen Reaktionsbedingungen eine Zwischenverbindung der Formel (IV) herstellt 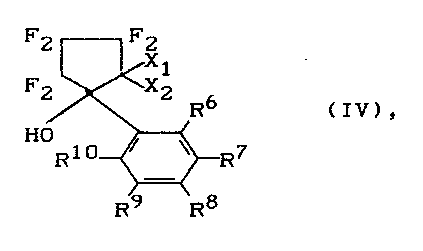
in der
X₁, X₂ und R⁶ bis R¹⁰ die bei den Formeln (II) und (III) angegebene Bedeutung haben.

Die Zwischenverbindungen der Formel (IV) können gegebenenfalls nach einer der oben angegebenen Methoden isoliert und gereinigt werden. Sie sind ebenfalls Gegenstand der vorliegenden Erfindung. Gegebenenfalls kann auf dieser Stufe auch eine Derivatisierung durchgeführt und so ein Arylrest mit nach Position und/oder Art anderen Substituenten erhalten werden. Durch Umsetzung eines Mols der gegebenenfalls derivatisierten, isolierten oder nicht isolierten Zwischenverbindung der Formel (IV) mit beispielsweise 1 bis 10 Mol einer anderen Arylverbindung der Formel (III) als der zuvor eingesetzten kann man dann unter den oben beschriebenen Reaktionsbedingungen Verbindungen der Formel (I) mit zwei verschiedenen Phenylringen A und B erhalten.

Selbstverständlich kann man auch Verbindungen der Formel (I), die theoretisch ohne Derivatisierung zugänglich sind, im Wege der Derivatisierung herstellen, indem man beispielsweise ein fluorhaltiges Cyclopentanon der Formel (II) zunächst mit einer der als bevorzugt oder besonders bevorzugt bezeichneten Arylverbindungen der Formel (III) umsetzt, und dann in den Ringen A und/oder B vorliegende Substituenten verändert, beispielsweise ein Wasserstoffatom in ein Fluor-, Chlor- oder Bromatom.

Im Hinblick auf den eingangs geschilderten Stand der Technik ist es außerordentlich überraschend, daß es mit dem erfindungsgemäßen Verfahren in Gegenwart von wasserfreier Flußsäure gelingt 1,1-Dicyclo-substituierte fluorhaltige Cyclopentane der Formel (I) in guten Ausbeuten und hohen Reinheiten und auch im technischen Maßstab herzustellen.

Die erfindungsgemäßen 1,1-Dicyclo-substituierten fluorhaltigen Cyclopentane der Formel (I) können als Zwischenprodukte zur Herstellung von Polycarbonaten, Polyestern und Polyestercarbonaten verwendet werden. Man kann dabei beispielsweise so verfahren, daß man Verbindungen der Formel (I), gegebenenfalls unter Zusatz anderer Bisphenole, mit aromatischen Dicarbonsäuredichloriden und/oder Phosgen, gegebenenfalls unter Zusatz von Katalysatoren, Kettenabbrechern, Farbverbesserern und/oder Verzweigungsmitteln, umsetzt. Solche Polyester, Polycarbonate und Polyestercarbonate zeichnen sich u.a. durch gute optische Eigenschaften aus. Sie sind von separaten eigenen Patentanmeldungen umfaßt, beispielsweise von der europäischen Patentanmeldung 90 123 879.0.

### Beispiele

### Beispiel 1

### Herstellung von 1,1-Di-(4-hydroxyphenyl)-octafluorcyclopentan

684 g (3,0 Mol) Octafluorcyclopentanon wurden in 1700 ml wasserfreier Flußsäure in einem 5 l fassenden Rührkessel aus V4A-Stahl mit Druckhaltung bei 5°C (Eiskühlung) vorgelegt. Nach Zugabe von 575 g (6,1 Mol) Phenol wurde die Reaktionstemperatur auf 25 bis maximal 40°C gesteigert und 6 Stunden lang gerührt. Anschließend wurde die Flußsäure bei maximal 100 mbar und 50°C abdestilliert und der gelblich-weiße Rückstand (ca. 1200 g) mit Wasser weitgehend säurefrei gewaschen. Das Rohprodukt hatte nach dem Trocknen einen Schmelzpunkt von 164 bis 167°C.

Zur weiteren Reinigung wurde das getrocknete Material in 2000 ml 5 %iger wäßriger Natronlauge gelöst und durch Einrühren von 10 %iger Salzsäure wieder ausgefällt. Nach Absaugen und Trocknen wurden 1140 g (2,86 Mol) analytisch reines Material mit einem Schmelzpunkt von 168 bis 170°C erhaltene. Die Ausbeute nach dem Umfällen betrug, bezogen auf eingesetztes Octafluorcyclopentanon, 95,3 %.

Das Massenspektrum zeigte bei m/e = 398 den Molpeak. Das ¹H-NMR-Spektrum zeigte Absorptionen bei δ = 6,74 ppm und 6,16 ppm, gemessen in d₄-Methanol gegen Tetramethylsilan als internen Standard, Die Hydroxylprotonen waren gegen Deuteronen ausgetauscht. Das ¹⁹F-NMR-Spektrum zeigte Absorptionen bei δ = -36,2 ppm und -48,5 ppm, gemessen gegen Trifluoressigsäure als externen Standard.

### Beispiel 2

### Herstellung von 1,1-Di-(4-hydroxyphenyl)-2,2-dichlorhexafluorcyclopentan

261 g (1,0 Mol) 2,2-Dichlorhexafluorcyclopentanon wurden in 500 ml wasserfreier Flußsäure in einem 1,7 l fassenden Rührkessel aus V4A-Stahl mit Druckhaltung bei 5°C (Eiskühlung) vorgelegt. Danach wurden 192 g (2,04 Mol) Phenol zugegeben und die Reaktionstemperatur auf 25 bis maximal 60°C gesteigert und anschließend 8 Stunden lang gerührt. Dann wurde die Flußsäure bei maximal 100 bar und 50°C abdestilliert und der bräunlich-graue Rückstand (ca. 430 g) mit Wasser weitgehend säurefrei gewaschen. Das Rohprodukt hatte nach dem Trocknen einen Schmelzpunkt von 159 bis 163°C.

Zur weiteren Reinigung wurde das Material in 800 ml 5 %iger wäßriger Natronlauge gelöst und durch Einrühren von 10 %iger Salzsäure wieder ausgefällt. Nach dem Absaugen und Trocknen wurden 395 g (0,92 Mol) Material mit gaschromatographisch festgestellter 95 %iger Reinheit und einem Schmelzpunkt von 163 bis 165°C erhalten. Die Ausbeute nach dem Umfällen betrug, bezogen auf eingesetztes 2,2-Dichlorhexafluorcyclopentanon 91,6 %.

Ein analytisch reines Material wurde durch Umkristallisation aus Trichlorethen unter Verwendung von Aktivkohle erhalten.

Das Massenspektrum zeigte bei m/e = 430 den Molpeak. Das ¹H-NMR-Spektrum zeigte Absorptionen bei δ = 3.4 ppm, 6.72 ppm und 7.11 ppm, gemessen in d₆-Dimethylsulfoxid gegen Tetramethylsilan als internen Standard, Das ¹⁹F-NMR-Spektrum zeigte Absorptionen bei δ = -29,6 ppm, - 34,0 ppm und -51,8 ppm, gemessen gegen Trifluoressigsäure als externen Standard.

### Beispiel 3

### Vergleichsbeispiel zu US-PS 3 388 097, Beispiel 1, d.h. im wesentlichen unter Verwendung von Essigsäure/Schwefelsäure anstelle von wasserfreier Flußsäure

130 g (0,5 Mol) 2,2-Dichlorhexafluorcyclopentanon wurden in einem 1 l-Glaskolben mit Rührer mit 105 g Phenol (1,12 Mol) und 100 ml Eisessig vorgelegt. Bei Raumtemperatur wurden dann innerhalb von 60 Minuten 300 ml 96 %ige Schwefelsäure zugetropft, wobei sich das Reaktionsgemisch deutlich erwärmte und bräunlich-schwarz verfärbte. Nach einer Nachrührzeit von 30 Minuten wurde das gesamte Reaktionsgemisch in 800 ml Eiswasser eingetragen. Es schied sich eine organische Phase ab, die nicht zur Kristallisation gebracht werden konnte.

Nach dem Aufnehmen in Methylenchlorid und Waschen mit Wasser verblieben nach dem Trocknen über Natriumsulfat und Abziehen des Methylenchlorids 120 g eines Öls, das durch Vergleich mit den Literaturdaten (J. Org. Chem. 33, 2692 (1968)) als das Hydrat des 2,2-Dichlorhexafluorcyclopentanons identifiziert wurde (Siedepunkt bei Normaldruck 107 bis 108°C, Schmelzpunkt 38 bis 40°C).

Das ¹⁹F-NMR-Spektrum des isolierten Materials zeigte Absorptionen bei δ = -37,6 ppm, -40,5 ppm und -41,8 ppm gemessen gegen Trifluoressigsäure als externen Standard, Dieses Spektrum ist völlig verschieden vom Spektrum des Produktes aus Beispiel 2 und identisch mit dem ¹⁹F-NMR-Spektrum einer authentischen Probe von 2,2-Dichlorhexafluorcyclopentanon-Hydrat.

### Beispiel 4

### Herstellung von 1-(4-Hydroxyphenyl)-1-(2',5'-dimethyl-4'-hydroxyphenyl)-octafluorcyclopentan und 1-(2,5-Di-methyl-4-hydroxyphenyl)-octafluorcyclopentan-1-ol

114 g (0,5 Mol) Octafluorcyclopentanon wurden in 400 ml wasserfreier Flußsäure in einem 1,3 l Rührkessel aus V4A-Stahl mit Druckhaltung bei 5°C (Eiskühlung) vorgelegt. Nach Zugabe von 73 g (0,6 Mol) 2,5-Dimethylphenol wurde die Reaktionstemperatur auf 70°C bis maximal 100°C gesteigert und 5 Stunden lang gerührt. Anschließend wurde die Flußsäure bei maximal 100 mbar und 80°C abdestilliert. Der verbleibende dunkelbraune, ölige Rückstand wurde in Methylenchlorid aufgenommen, filtriert, mit Wasser säurefrei gewaschen und über Natriumsulfat getrocknet. Nach Abziehen des Lösungsmittels wurde im Ölpumpenvakuum destilliert. Nach einem geringen Vorlauf von nicht umgesetztem 2,5-Dimethylphenol wurden 126 g (0,38 Mol) 1-(2,5-Dimethyl-4-hydroxyphenyl)-octafluorcyclopentan-1-ol mit einem Siedepunkt bei 0,03 mbar von 80 bis 83°C erhalten. Das entspricht 72 % der Theorie.

Das ¹H-NMR-Spektrum zeigte Absorptionen bei δ = 2,16 ppm, 2,22 ppm, 6,71 ppm und 7,07 ppm, gemessen in Deuterochloroform gegen Tetramethylsilan. Das ¹⁹F-NMR-Spektrum zeigte Absorptionen bei δ = -31,0 ppm, - 40,5 ppm, -47,4 ppm, -49,7 ppm und -51,9 ppm gemessen in Deuterochloroform gegen Trifluoressigsäure als externen Standard.

Das so erhaltene Monoaddukt wurde anschließend in 200 ml wasserfreier Flußsäure in einen 0,5 l Rührkessel aus V4A-Stahl mit Druckhaltung mit 20 g (0,21 Mol) Phenol bei 80°C bis maximal 100°C 8 Stunden lang gerührt. Nach dem Abdestillieren der Flußsäure bei maximal 100 mbar und 80°C wurde der verbleibende Rückstand in 200 ml 5 %iger wäßriger Natronlauge gelöst und mit 10 %iger wäßriger Salzsäure ausgefällt. Nach dem Trocknen bei maximal 110°C wurden 73 g (0,17 Mol) des unsymmetrischen Bisadduktes erhalten, was 85,6 % der Theorie entspricht. Der Schmelzpunkt lag bei 183 bis 185°C. Das Massenspektrum zeigte bei m/e = 426 den Molpeak.

Bei einer Wiederholung dieses Beispiels ohne Zwischenisolierung des 1-(2,5-Dimethyl-4-hydroxyphenyl)-octafluorcyclopentan-1-ols wurde 1-(4-Hydroxyphenyl)-1-(2',5'-dimethyl-4'-hydroxyphenyl)-octafluorcyclopentan in geringfügig schlechterer Reinheit und Ausbeute als im zweistufigen Verfahren erhalten.

### Beispiel 5

### Herstellung von 1,1-Di-(3,6-dimethyl-4-hydroxyphenyl)octafluorcyclopentan

In einem 1,3 l V4A-Rührkessel mit Druckhaltung wurden 400 ml wasserfreie Flußsäure eingebracht und bei 5°C unter Eiskühlung 114 g (0,5 Mol) Octafluorcyclopentanon zugefügt. Danach wurden weiterhin unter Druckhaltung 125 g (1,02 Mol) 2,6-Dimethylphenol hinzugefügt und die Temperatur auf 50°C bis maximal 60°C erhöht und die Mischung 8 Stunden lang gerührt. Danach wurde die Fluorwasserstoffsäure abdestilliert bei maximal 100 mbar und 80°C. Der zurückbleibende dunkelbraune Rückstand wurde mit Wasser säurefrei gewaschen. Es verblieb ein rohes Produkt in einer Menge von 220 g.

Zur weiteren Reinigung wurde das Produkt in 500 ml 5 gew.-%iger wäßriger Natronlauge gelöst und erneut ausgefällt durch Einrühren in 10 gew.-%ige wäßrige Salzsäure. Nach dem Abfiltrieren und Trocknen verblieben 215 g (0,47 Mol) Produkt mit einem Schmelzpunkt von 178 bis 181°C. Die Ausbeute nach der Umfällung betrug 84,7 % bezogen auf eingesetztes Octafluorcyclopentanon.

Das ¹H-NMR-Spektrum zeigte Absorptionen bei δ = 3,33 ppm, 6,88 ppm und 8,6 ppm, gemessen in d₆-Dimethylsulfoxid gegen Tetramethylsilan als internen Standard. Das ¹⁹F-NMR-Spektrum zeigte Absorptionen bei δ = -33,9 ppm und -46,3 ppm, gemessen in d₆-Dimethylsulfoxid gegen Trifluoressigsäure als externen Standard.

## Patentansprüche

1. 1,1-Dicyclo-substituierte fluorhaltige Cyclopentane der Formel (I) 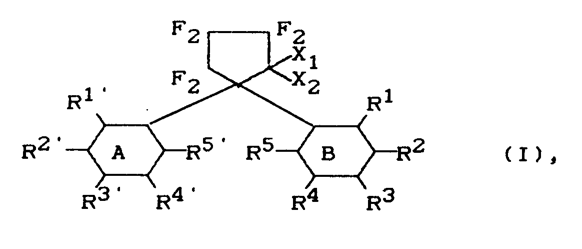 in der
X₁ und X₂ unabhängig voneinander für Fluor oder Chlor stehen,
A und B je für einen Phenylring stehen,
R¹, R^{1'}, R², R^{2'}, R⁴, R^{4'}, R⁵ und R^{5'} unabhängig voneinander für Wasserstoff, C₁-bis C₄-Alkyl, Fluor, Chlor oder Brom stehen und
R³ und R^{3'} je für Hydroxy stehen.

2. 1,1-Dicyclo-substituierte fluorhaltige Cyclopentane gemäß Anspruch 1, dadurch gekennzeichnet, daß in Formel (I)
A und B je einen Phenylrest darstellen und
R¹ und R^{1'} gleich sind und für Wasserstoff oder C₁-bis C₄-Alkyl stehen,
R² und R^{2'} gleich sind und für Wasserstoff, C₁- bis C₄-Alkyl, Fluor, Chlor oder Brom stehen,
R³ und R^{3'} gleich sind und für OH stehen,
R⁴ und R^{4'} gleich sind und für Wasserstoff oder C₁-bis C₄-Alkyl stehen und
R⁵ und R^{5'} für Wasserstoff stehen.

3. 1,1-Dicyclo-substituierte fluorhaltige Cyclopentane gemäß Anspruch 1, dadurch gekennzeichnet, daß in Formel (I) X₁ und X₂ für Fluor stehen, A und B je einen Phenylrest darstellen, die jeweils nicht besonders angeführten Reste R¹ bis R⁵ und R^{1'} bis R^{5'} für Wasserstoff stehen und
a) R³ = R^{3'} = OH,
b) R¹ = R^{1'} = R⁴ = R^{4'} = Methyl und R³ = R^{3'} = OH,
c) R² = R^{2'} = Methyl und R³ = R^{3'} = OH und
d) R² = R^{2'} = Chlor und R³ = R^{3'} = OH sind.

4. Verfahren zur Herstellung von 1,1-Dicyclo-substituierten fluorhaltigen Cyclopentanen des Anspruchs 1, dadurch gekennzeichnet, daß man fluorhaltige Cyclopentanone der Formel (II) 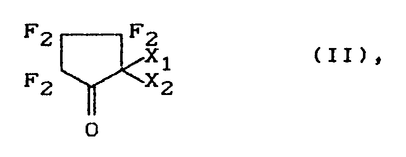 in der
X₁ und X₂ die in Anspruch 1 angegebene Bedeutung haben,
mit mindestens der 2-fachen molaren Menge einer oder mehrerer Arylverbindungen der Formel (III) 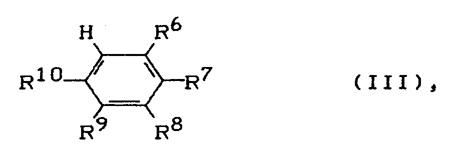 in der
R⁶ bis R¹⁰ die in Anspruch 1 für R¹ bis R⁵ angegebene Bedeutung haben
in Gegenwart von wasserfreier Flußsäure umsetzt, wobei die Menge wasserfreie Flußsäure so gewählt wird, daß deren Konzentration im Verlauf der Reaktion nicht unter 70 Gew.-% absinkt.

5. Verfahren nach Anspruch 4, dadurch gekennzeichnet, daß man auf 1 Mol eines fluorhaltigen Cyclopentanons der Formel (II) 2 bis 10 Mole einer Arylverbindung der Formel (III) einsetzt.

6. Verfahren nach Ansprüchen 4 und 5, dadurch gekennzeichnet, daß die Reaktionstemperatur im Bereich von 20 bis 150°C liegt.

7. Verfahren nach Ansprüchen 4 bis 6, dadurch gekennzeichnet, daß man Verbindungen der Formel (I), bei denen A und B zwei verschiedene Phenylringe darstellen, herstellt, indem man zunächst bezogen auf 1 Mol einer fluorhaltigen Cyclopentanons der Formel (II) 0,9 bis 1,1 Mol einer Arylverbindung der Formel (III) einsetzt und so zunächst eine Zwischenverbindung der Formel (IV) erhält 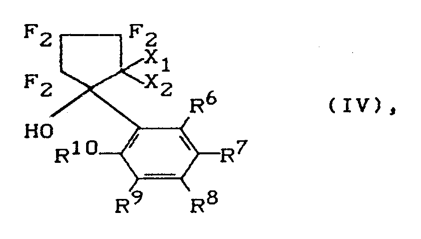 in der
X₁, X₂ und R⁶ bis R¹⁰ die bei den Formeln` (11) und (III) angegebene Bedeutung haben,
und anschließend die Zwischenverbindung mit der 1 bis 10-fachen molaren Menge einer anderen Arylverbindung der Formel (III) als der zuvor eingesetzten umsetzt.

8. Verbindungen der Formel (IV) 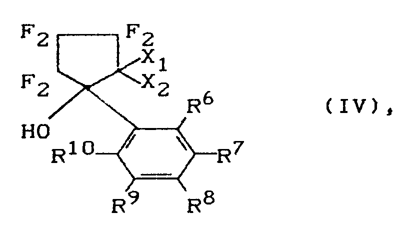 in der
X₁ und X₂ die in Anspruch 1 angegebene Bedeutung haben und
R⁶ bis R¹⁰ die in Anspruch 4 angegebene Bedeutung haben.

## Claims

1. 1,1-Bicyclo-substituted fluorine-containing cyclopentanes of the formula (I) 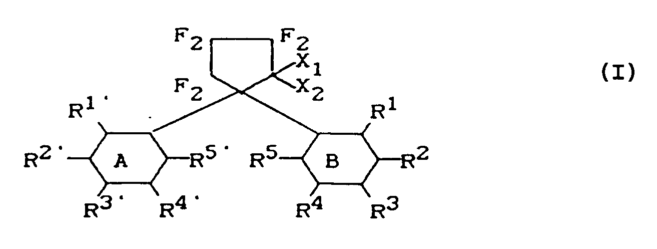 in which
X₁ and X₂ independently of one another represent fluorine or chlorine,
A and B each represent a phenyl ring,
R¹, R^{1',} R², R^{2'}, R⁴, R^{4'}, R⁵ and R^{5'} independently of one another represent hydrogen, C₁- to C₄-alkyl, fluorine, chlorine or bromine and
R³ and R^{3'} each represent hydroxyl.

2. 1,1-Bicyclo-substituted fluorine-containing cyclopentanes according to Claim 1, characterized in that, in formula (I)
A and B each represent a phenyl radical and
R¹ and R^{1'} are identical and represent hydrogen or C₁- to C₄-alXyl,
R² and R^{2'} are identical and represent hydrogen, C₁-to C₄-alkyl, fluorine, chlorine or bromine,
R³ and R^{3'} are identical and represent OH,
R⁴ and R^{4'} are identical and represent hydrogen or C₁- to C₄-alkyl and
R⁵ and R^{5'} represent hydrogen.

3. 1,1-Bicyclo-substituted fluorine-containing cyclopentanes according to Claim 1, characterized in that, in formula (I), X₁ and X₂ represent fluorine, A and B each represent a phenyl radical, the radicals R¹ to R⁵ and R^{1'} to R^{5'} which are not especially mentioned represent hydrogen and
a) R³ = R^{3'} = OH,
b) R¹ = R^{1'} = R⁴ = R^{4'} = methyl and R³ = R^{3'} = OH,
c) R² = R^{2'} = methyl and R³ = R^{3'} = OH and
d) R² = R^{2'} = chlorine and R³ = R^{3'} = OH.

4. Process for the preparation of 1,1-bicyclo-substituted fluorine-containing cyclopentanes of Claim 1, characterized in that fluorine-containing cyclopentanones of the formula (II) 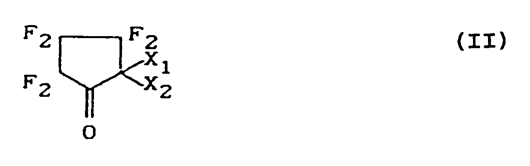 in which
X₁ and X₂ have the meaning indicated in Claim 1
are reacted with at least twice the molar amount of one or more aryl compounds of the formula (III) 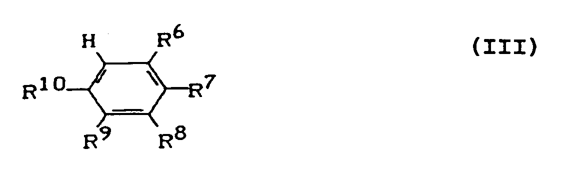 in which
R⁶ to R¹⁰ have the meaning indicated in Claim 1 for R¹ to R⁵,
in the presence of anhydrous hydrofluoric acid, the amount of anhydrous hydrofluoric acid being chosen such that its concentration in the course of the reaction does not fall below 70 % by weight.

5. Process according to Claim 4, characterized in that 2 to 10 moles of an aryl compound of the formula (III) are employed per mole of a fluorine-containing cyclopentanone of the formula (II).

6. Process according to Claims 4 and 5, characterized in that the reaction temperature is in the range from 20 to 150°C.

7. Process according to Claims 4 to 6, characterized in that compounds of the formula (I), in which A and B represent two different phenyl rings, are prepared by first employing 0.9 to 1.1 moles of an aryl compound of the formula (III) relative to 1 mole of a fluorine-containing cyclopentanone of the formula (II) and thus initially obtaining an intermediate compound of the formula (IV) 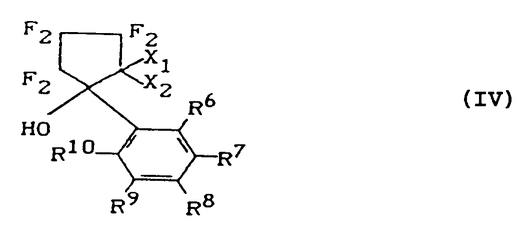 in which
X₁, X₂ and R⁶ to R¹⁰ have the meaning indicated for the formulae (II) and (III),
and subsequently reacting the intermediate compound with 1 to 10 times the molar amount of an aryl compound of the formula (III) other than that previously employed.

8. Compounds of the formula (IV) 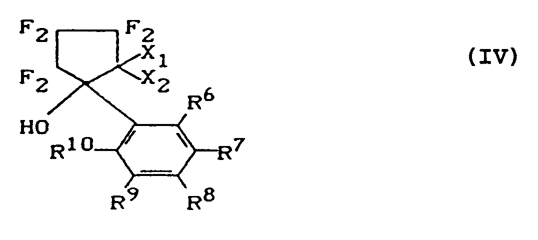 in which
X₁ and X₂ have the meaning indicated in Claim 1 and
R⁶ to R¹⁰ have the meaning indicated in Claim 4.

## Revendications

1. Cyclopentanes fluorés 1,1-dicyclo-substitués répondant à la formule (I) 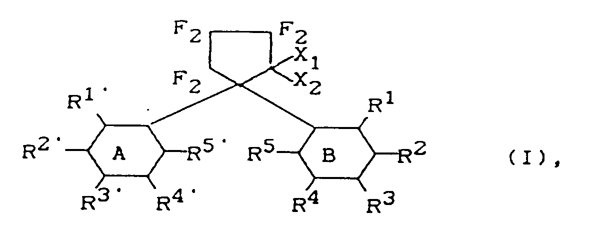 dans laquelle
X₁ et X₂ représentent, chacun indépendamment l'un de l'autre, un atome de fluor ou un atome de chlore,
A et B représentent chacun un noyau phényle,
R¹, R^{1'}, R², R^{2'}, R⁴, R^{4'}, R⁵ et R^{5'} représentent, chacun indépendamment l'un de l'autre, un atome d'hydrogène, un groupe alkyle en C₁-C₄, un atome de fluor, un atome de chlore ou un atome de brome, et
R³ et R^{3'} représentent chacun un groupe hydroxyle.

2. Cyclopentanes fluorés 1,1-dicyclo-substitués selon la revendication 1, caractérisés en ce que, dans la formule (I)
A et B représentent chacun un radical phényle et
R¹ et R^{1'} sont identiques et représentent un atome d'hydrogène ou un groupe alkyle en C₁-C₄,
R² et R^{2'} sont identiques et représentent un atome d'hydrogène, un groupe alkyle en C₁-C₄, un atome de fluor, un atome de chlore ou un atome de brome,
R³ et R^{3'} sont identiques et représentent OH,
R⁴ et R^{4'} sont identiques et représentent un atome d'hydrogène ou un groupe alkyle en C₁-C₄, et
R⁵ et R^{5'} représentent un atome d'hydrogène.

3. Cyclopentanes fluorés 1,1-dicyclo-substitués selon la revendication 1, caractérisés en ce que, dans la formule (I), X₁ et X₂ représentent un atome de fluor, A et B représentent chacun un radical phényle, les radicaux R¹ à R⁵ et R^{1'} à R^{5'} qui ne sont pas respectivement désignés de manière spécifique représentent un atome d'hydrogène, et
a) R³ = R^{3'} = OH,
b) R¹ = R^{1'} = R⁴ = R^{4'} = un groupe méthyle et R³ = R^{3'} = OH,
c) R² = R^{2'} = un groupe méthyle et R³ = R^{3'} = OH, et
d) R² = R^{2'} = un atome de chlore et R³ = R^{3'} = OH.

4. Procédé pour la préparation de cyclopentanes fluorés 1,1-dicyclo-substitués selon la revendication 1, caractérisé en ce qu'on fait réagir des cyclopentanones fluorées de formule (II) 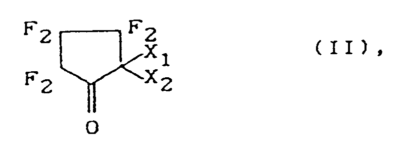 dans laquelle
X₁ et X₂ ont la signification indiquée à la revendication 1,
avec au moins la quantité 2 fois molaire d'un ou de plusieurs composés arylés de formule (III) 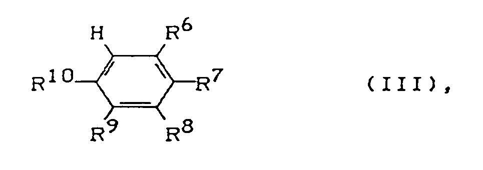 dans laquelle
R⁶ à R¹⁰ ont la signification indiquée pour R¹ à R⁵ dans la revendication 1,
en présence d'acide fluorhydrique anhydre, la quantité en acide fluorhydrique anhydre étant sélectionnée de telle sorte que sa concentration ne tombe pas en dessous de 70% en poids au cours de la réaction.

5. Procédé selon la revendication 4, caractérisé en ce que, pour l mole d'une cyclopentanone fluorée de formule (II), on met en oeuvre de 2 à 10 moles d'un composé arylé de formule (III).

6. Procédé selon les revendications 4 et 5, caractérisé en ce que la température réactionnelle se situe dans le domaine de 20 à 150°C.

7. Procédé selon les revendications 4 à 6, caractérisé en ce qu'on prépare des composés de formule (I) dans lesquels A et B représentent deux noyaux phényle différents, en mettant d'abord en oeuvre, rapportée à 1 mole d'une cyclopentanone fluorée de formule (II), de 0,9 à 1,1 mole d'un composé arylé de formule (III) et en obtenant ainsi d'abord un composé intermédiaire de formule (IV) 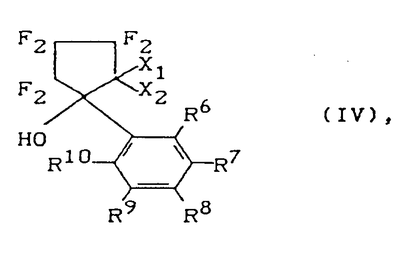 dans laquelle
X₁, X₂ et R⁶ à R¹⁰ ont la signification indiquée dans les formules (II) et (III),
et ensuite, en faisant réagir le composé intermédiaire avec une quantité de 1 à 10 fois molaire d'un composé arylé de formule (III) différent de celui mis en oeuvre précédemment.

8. Composés de formule (IV) 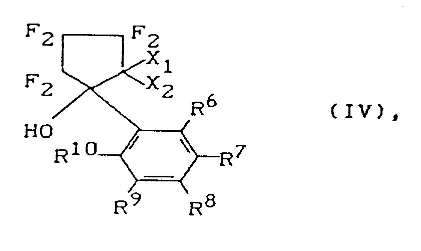 dans laquelle
X₁ et X₂ ont la signification indiquée à la revendication 1, et
R⁶ à R¹⁰ ont la signification indiquée à la revendication 4.
